# EUROPEAN PATENT SPECIFICATION

(11) **EP 2 351 493 B1**
(45) Date of publication and mention of the grant of the patent: **04.11.2015**
(21) Application number: 08875583.0
(22) Date of filing: 10.09.2008
(51) Int. Cl.: A23B 4/20

(54) **COMPOSITION BASED ON CONJUGATED LINOLEIC ACID FOR CANNED PRODUCTS COMPRISING AT LEAST ONE TUNA**
ZUSAMMENSETZUNG AUF BASIS VON KONJUGIERTER LINOLSÄURE FÜR KONSERVENPRODUKTE, DIE ZUMINDEST THUNFISCH ENTHALTEN
COMPOSITION À BASE D'ACIDE LINOLÉIQUE CONJUGUÉ POUR CONSERVES COMPRENANT AU MOINS UN THONIDÉ

(43) Date of publication of application: 03.08.2011
(73) Proprietor: Salica, Industria Alimentaria, S.A., 48370 Bermeo (ES)
(72) Inventor: PAGAZAURTUNDUA ZABALA, Marta, E-48394 Murueta (ES); AYO MARTÍNEZ, Josune, E-48009 Bilbao (ES); SÁNCHEZ-ALONSO, Isabel, E-28909 Perales del Río (Getafe) (ES)
(74) Representative: Ezcurra Zufia, Maria Antonia
(86) International application number: PCT/ES2008/070171
(87) International publication number: WO 2010/029191

(56) References cited:
- US-A1- 2007 111 292
- MARTIN D ET AL: "Partial replacement of pork fat by conjugated linoleic acid and/or olive oil in liver pates: Effect on physicochemical characteristics and oxidative stability." MEAT SCIENCE 80 (2) 496-504 2008 DEPARTMENT OF FOOD SCIENCE, UNIVERSIDAD DE EXTREMADURA, FACULTAD DE VETERINARIA, AVDA. UNIVERSIDAD S/N, 10071 CACERES, SPAIN. TEL. +34 927 257123. FAX +34 927 257110. E-MAIL DIMAGA(A)UNEX.ES, 2008, XP002546135
- HA Y L ET AL: "INHIBITION OF BENZO(A)PYRENE-INDUCED MOUSE FORESTOMACH NEOPLASIA BY CONJUGATED DIENOIC DERIVATIVES OF LINOLEIC ACID" CANCER RESEARCH, AMERICAN ASSOCIATION FOR CANCER RESEARCH, BALTIMORE, MD., US, vol. 50, no. 4, 15 February 1990 (1990-02-15), pages 1097-1101, XP001093870 ISSN: 0008-5472
- RISERUS U ET AL: "CLA AND BODY WEIGHT REGULATION IN HUMANS" LIPIDS, CHAMPAIGN, IL, US, vol. 38, no. 2, 1 February 2003 (2003-02-01), pages 133-137, XP009021027 ISSN: 0024-4201
- GAULLIER J-M ET AL: "Clinical trial results support a preference for using CLA preparations enriched with two isomers rather than four isomers in human studies" LIPIDS, CHAMPAIGN, IL, US, vol. 37, no. 11, 1 November 2002 (2002-11-01), pages 1019-1025, XP008111800 ISSN: 0024-4201
- BUTZ D E ET AL: "t10,c12 conjugated linoleic acid induces compensatory growth after immune challenge" JOURNAL OF NUTRITIONAL BIOCHEMISTRY, BUTTERWORTH PUBLISHERS, STONEHAM, GB, vol. 17, no. 11, 1 November 2006 (2006-11-01), pages 735-741, XP024960882 ISSN: 0955-2863 [retrieved on 2006-11-01]
- YANG LIN ET AL: "Stability of conjugated linoleic acid isomers in egg yolk lipids during frying" FOOD CHEMISTRY, vol. 86, no. 4, August 2004 (2004-08), pages 531-535, XP002546137 ISSN: 0308-8146
- YANG LIN ET AL: "Oxidative stability of conjugated linoleic acid isomers" JOURNAL OF AGRICULTURAL AND FOOD CHEMISTRY, vol. 48, no. 8, August 2000 (2000-08), pages 3072-3076, XP002546136 ISSN: 0021-8561
- MANNING BRUCE B ET AL: "Enrichment of channel catfish (Ictalurus punctatus) fillets with conjugated linoleic acid and omega-3 fatty acids by dietary manipulation" AQUACULTURE, vol. 261, no. 1, November 2006 (2006-11), pages 337-342, XP002546138 ISSN: 0044-8486
- HAH K H ET AL: "Effect of substituted conjugated linoleic acid on meat qualities, lipid oxidation and residual nitrite content in emulsion-type sausage." ASIAN-AUSTRALASIAN JOURNAL OF ANIMAL SCIENCES 19 (5) 744-750 2006 CORRESPONDENCE (REPRINT) ADDRESS, S. T. JOO, DIV. OF APPLIED LIFE SCI., GRADUATE SCH., GYEONGSANG NAT. UNIV., JINJU, KOREA. TEL. +82 55 751 5515. FAX +82 55 756 7171. E-MAIL STJOO(A, 2006, XP008111856

## Description

### OBJECT OF THE INVENTION

The object of this invention is a composition of the two bioactive isomers of conjugated linoleic acid (cis-9, trans-11 and trans-10, cis-12) at 50:50 at a proportion of 7.5% to 15% in olive oil, which remains stable through the sterilization process and storage for canned products including tuna fish, retaining its properties for the reduction of body fat and as a lipid metabolism activator.

### BACKGROUND OF THE INVENTION

Linoleic acid (9.12-octadecadienoic acid) is a fatty acid with 18 carbon atoms and two unsaturated or double bonds located in positions 9 and 12 respectively. Chains C1-C8 and C13-C18 are in cis position relative to the double bonds.

Linoleic acid is an essential fatty acid, very abundant in vegetable oils.

Conjugated linoleic acid, which in this paper will be abbreviated as CLA, is a generic name for a group of linoleic acid isomers. Of the large number of isomers of CLA, it is especially cis-9, trans-11 and trans-10, cis-12 that are primarily responsible for the beneficial effects on the organism, with body fat reduction and lipid metabolism activator properties. Herein they will be called bioactive CLA isomers.

Whigham et al (2007) concluded that CLA administered in doses of 3.2 g / day was effective since it produces a significant decrease of body fat in humans.

Gaullier et al (Gaullier et al., 2004) demonstrated that preparations of CLA highly enriched with cis-9, trans-11 and trans-10, cis-12 at 50:50 are correct for human consumption in terms of effectiveness in reducing body fat. The estimated effective dose was 1.7 g - 3.4g of these isomers of CLA per day.

The major source of CLA in humans is milk and its derivatives. Other sources through diet are ruminant meat and some vegetable oils. Changes during recent years in animal feed (feed consumption rather than grass), combined with current trends of lower consumption of milk and milk products have drastically reduced the amount of CLA acquired by humans through diet.

The average intake of CLA in the population depends largely on the type of diet, depending on whether foods that include it are eaten.Thus, for example, the average intake in the European population is 350-430 mg/day and in the U.S. population 150-200 mg/day.

All this tends to confirm that in order to expect a body fat reduction effect in the organism and an increase in lipid metabolism, that may contribute to the fight against obesity, it will be useful to provide an extra contribution of CLA through diet.

The incorporation of CLA to the diet with an appropriate vehicle is therefore of great interest.

The problem that arises when incorporating bioactive isomers in the diet through foods that include them is that until now it has not been possible to obtain a composition in which, after processing through the high temperature treatments to which certain foods are subjected, the bioactive isomers remain stable without being degraded.

There are several studies on the stability of CLA isomers. Yurawecz, Hood, Mosoba, Roach and Yu (1995) have shown that CLA in its purest form is rapidly degraded to furan (fatty acids) when heated in air.

Other studies on the stability of the CLA isomers in the form of free fatty acids have shown, using a combination of gas-liquid chromatography (GLC) and Ag-HPLC (High Performance Liquid Chromatography Resolution and silver ion) that CLA is unstable.

In the study by Yang et al. (2000), 12 CLA isomers were subjected to 50[deg.] C for 110 hours in the air to see the effect of oxidative stability, using the above detection technique: GLC and Ag-HPLC. The results concluded that the CLA in free form was extremely unstable, similar to docosahexanenoic acid (Chen et al., 1997) and that it has an oxidation rate considerably higher than that of linoleic acid and arachidonic acid.

In fact, Yurawecz et al. (1995) reported that when it was oxidized in the air, CLA was rapidly decomposed to furan fatty acids.

The 4 c, c-CLA isomers were the most unstable followed by another 4 c, t-CLA isomers - unlike the other 4 t, t-CLA isomers, which were relatively stable under the same experimental conditions. In total, over 80% oxidized after 110 hours in the air at 50 [deg.] C.

Minemoto et al. (2003) showed that the two bioactive CLA isomers are more susceptible to oxidation than linoleic acid (LA), and are similar to docosahexaenoic acid (DHA). They also showed that CLA is extremely unstable both in the form of free fatty acid (FFA) and in the form of triacylglycerol (TAG). The study was conducted at different temperatures: 50-80 [deg.] C for both isomers with the result that the isomer 10t, 12c oxidized faster than 9c, 11t.

This study showed that if CLA is incorporated in a food and subjected to processing, the CLA would be degraded, thus losing its beneficial body fat reduction properties.

In addition to the influence of the heat treatment on the stability of CLA during processing and storage, studies show that the behavior of CLA depends additionally on and is conditioned by the food matrix in which it is found, as there are interactions between the components present in the food that cause the degradation of CLA.

When CLA isomers were added to a meat emulsion (Hur et al., 2003), the total CLA content increased, while the effect of cold storage (4 [deg.] C) for 14 days in a meat emulsion with CLA barely had an effect on the CLA concentration. In terms of color it was improved, due to the inhibition of lipid and myoglobin oxidation, which was corroborated with a decrease in TBARS levels in samples with CLA.

However, in CLA-enriched milk, pasteurization and homogenization (HTST) caused a significant decrease of the 9c, 11T and other minor isomers (Campbell et al., 2003). After being kept refrigerated for 2 weeks isomers 9c, 11T were stable. But after 3 weeks of refrigeration, both isomers 9c, 11T and 10c, 12t decreased significantly. As for the sensory characteristics, the CLA-enriched milk had a grass/vegetable flavor, but by adding chocolate or strawberry flavoring masking is achieved.No differences in viscosity were observed with the control sample, and the milk had a less white color.

It is known in the state of the art, additionally to the already mentioned documents, the following ones:
- US 200711292 A1 which discloses structures lipid mixtures
- MARTIN D ET AL: "Partial replacement of pork fat by conjugated linoleic acid and/or olive oil in liver pates: Effect on physicomchemical characteristics and oxidative stability" MEAT SCIENCE 8.0 (2) 496-504-2008 DEPARTMENT OF FOOT SERVICE SCIENCE, UNIVERSIDAD DE EXTREMADURA, FACULTAD DE VETERINARIA. XP002546135
- HA Y L ET AL: "Inhibition of Benzo(A)pyrene-induced mouse forestomach neoplasia by conjugated dienoic derivatives of linoleic acid" Cancer research, American association for cancer research, Baltimore, MD, US vol 50, no 4, 15 February 1990 (1990-02-05) PAGES 1097-1101, XP001093870 ISSN:0008-5472

Consequently, the rate of oxidative degradation of CLA is determined by many factors, such as the activation of energy, the potential of oxide-reduction, temperature, available oxygen and the matrix in which it is located.

Thus, CLA cannot be added to certain foods since after the processing that they undergo, they reach the final consumer in degraded form, losing their beneficial body fat reduction properties and lipid metabolism activation.

In the present invention this technical problem is solved, since through the composition that is the object of the present invention which comprises a mixture of bioactive isomers and olive oil rich in antioxidants, in a canned food matrix including tuna fish, oxidative stability is improved for bioactive CLA isomers after the sterilization process and storage of those cans.

Thus, the oxidative stability obtained of CLA mixed with oil and subjected to heat processes finds an application in fish products including tuna fish that are subjected to sterilization (116 [deg.] C for 45 minutes.)

The object of this invention is a composition based on conjugated linoleic acid which is stable during processing and storage in canned foods including tuna fish. The composition of the present invention is an improvement over those already known as it does not degrade over time (long-term storage) or during the heat treatment that takes place during the processing of the cans (116[deg.] C for 45 minutes) while the bioactive CLA isomers are stable in the tuna's food matrix.

In particular, this invention relates to a composition based on conjugated linoleic acid which includes a ratio between 7.5 and 15% of the bioactive isomers of CLA at 50:50 in olive oil which remains stable during processing and storage of canned foods including tuna fish, so that the composition has the expected beneficial effect on body fat reduction and activation of lipid metabolism after regular consumption of such canned foods.

It also provides a stable composition in a food matrix including tuna fish that presents no sensory differences compared to canned tuna preparations that do not include CLA, since, according to hedonic analysis, all samples are considered as good, with the tasters detecting no differences in the parameters considered (appearance, aroma, flavor and texture) in the samples with and without CLA.

### DESCRIPTION OF THE INVENTION

There is known degradation of bioactive isomers of conjugated linoleic acid at high temperatures, and it has been found that these remain stable in a canned tuna-based product.

Degradation in the content of bioactive CLA isomers (cis-9, trans-11 and trans-10, cis-12, c18: 2) was studied in a can of tuna due to sterilization and storage.

For this an appropriate analytical methodology for the identification and quantification of bioactive CLA isomers was used, with benchmarks identified by retention time in gas chromatography with an FID detector.

Furthermore, interactions among the ingredients that make up the new food preparation have been taken into account, and it has been guaranteed that the mixture has succeeded in improving the stability of the incorporated bioactive isomers of the CLA.

The mixture of CLA with olive oil rich in antioxidants in the composition set out in this invention keeps the bioactive CLA isomers stable, preventing oxidative degradation, at room temperatures for processing the cans, sterilization at 116 [deg.] C for 45 minutes, stable upon storage and stable in the matrix of canned tuna.

Thus, the composition which is the object of the invention is a composite based on conjugated linoleic acid which includes a ratio between 7.5 and 15% of the bioactive isomers of CLA at 50:50 in olive oil that remains stable during processing and storage of canned goods comprising tuna fish.

With the composition of the invention, the losses in the content of bioactive isomers of CLA (cis-9, trans-11 and trans-10, cis-12, C18: 2) were minimized after the sterilization process, distributed equally for both isomers.

After the sterilization process, the cans with the tuna with olive oil and CLA were stored at room temperature in order to determine the content of bioactive CLA isomers in the can of tuna during storage.

During the first nine months of storage no reduction was seen in the content of cis-9, trans-11 and trans-10, cis-12 C18: 2.

This ensures that the can of tuna incorporates the effective amount of bioactive isomers of CLA to achieve an improvement in reducing body fat after consumption of the product.

It is thus possible to make a composition that remains stable during processing (sterilization at 116 [deg.] C for 45 minutes) and a composition that remains stable during storage, without altering or degrading these isomers, while keeping their properties for reducing body mass.

In different trials, CLA content variability from one batch to another was also evaluated, to verify that the amount of CLA was repeated. In all trials, the CLA content in the cans was enough to reach the range in which these isotopes of CLA have beneficial effects on health in humans.

Additionally, the potential of canned tuna with CLA has been demonstrated through a nutritional intervention trial where its impact on reducing body fat was evaluated in volunteers who consumed one can per day of the new product within a calorie restricted diet.

A nutritional intervention trial was conducted with the purpose of demonstrating the effect on the reduction of body fat after daily consumption of canned tuna incorporating conjugated linoleic acid.

As a result of the nutritional intervention study in humans it was concluded that the consumption of the canned product including tuna fish and containing a composition between 7.5 and 15% of CLA in a 50:50 ratio of the two bioactive isomers , cis-9, trans-11 and trans-10, cis-12, in olive oil for 10 weeks was able to induce favorable changes in body composition in overweight and obese individuals subjected to controlled energy restriction, detected through DEXA.

The DEXA method of body composition analysis was able to detect significant differences in the percentage of total body fat and truncal fat (significant reduction in both measures in participants who received CLA), compared to the participants who did not take CLA.

In summary, the present invention relates to a composition with conjugated linoleic acid for canned products including tuna fish characterized as including between 7.5 and 15% of the bioactive isomers of CLA at 50:50 in olive oil.

Thus, the resulting formulation makes the CLA, which may be degraded at high temperatures and over long periods of storage, stable in the final product developed.

### DESCRIPTION OF THE DRAWINGS

This report is complemented by a set of drawings which illustrate one preferred example and are in no way limitations for the invention.

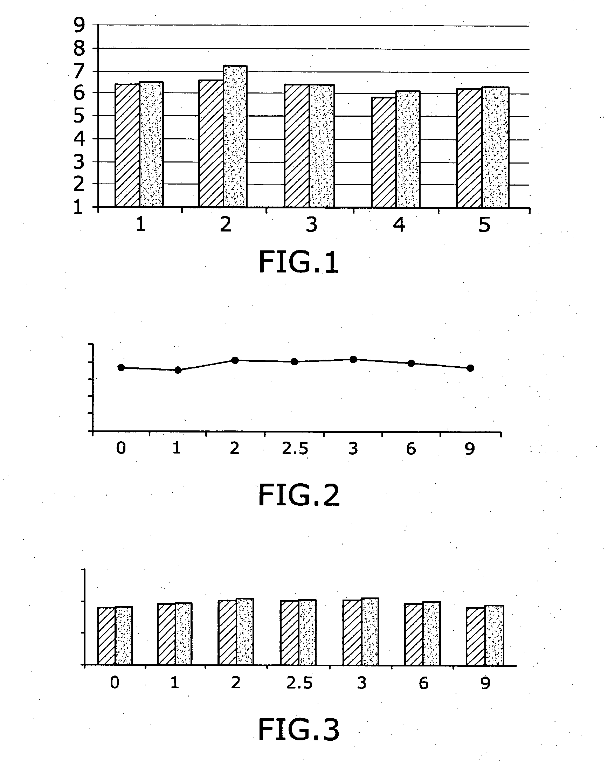

Figure 1 shows a graph of the sensory evaluation of samples of canned tuna with olive oil and CLA. The ordinate represents the hedonic scale with values from 1 to 9, wherein the samples with a higher score were better accepted. The horizontal axis represents the appearance with a 1, the scent with a 2, the taste with a 3, the texture with a 4 and the average with a 5.
   The striped column represents the canned tuna in olive oil as a means of coverage. The column of dots represents the canned tuna in olive oil with CLA as a means of coverage.
Figure 2 shows a graph which represents the total content of the two bioactive isomers of CLA in grams of bioactive isomer / can after processing and throughout the storage period at room temperature. The horizontal axis represents the months of storage. Zero time marks the beginning of the study and therefore these are products containing 100% of the effective dose of CLA per can.
Figure 3 shows a graph which represents the contents of each of the bioactive isomers of CLA in g / can separately after processing and throughout the storage period.

The striped column represents the contents of the bioactive isomer cis-9, trans-11, and the column in dots represents the bioactive isomer trans-10, cis-12.35. As in the previous chart, the abscissa represents the months of storage.

### DETAILED DESCRIPTION OF THE INVENTION

For the determination of the CLA-based composition that is the object of the invention, laboratory experiments have been conducted that show how their properties exceed those obtained in the state of the technique, as a composition is achieved that remains stable during the processing and storage of canned tuna.

To perform these experiments, samples of canned Thunnus obesus were prepared. The covering oil selected was olive oil, high in antioxidants.

The process of preparing the cans of tuna in the industrial plant was developed in the following manner.

The composition between 7.5 and 15% of the bioactive isomers of CLA at 50:50 in olive oil was added to each can including one tuna; water was added and the canning industrial process was continued.

The cans are then sent off to the autoclave. The sterilization process was carried out in an autoclave for 45 minutes at 116 [deg.] C.

This olive oil has a composition of 97% refined olive oil and 3% extra virgin and complies with the regulations set out in the standard CE-2568/91 and its subsequent amendments. It is suitable for the production of canned fish of good quality, free of chlorinated products (Perchloroethylene, Trichloroethylene and chloroform) and esterified oils or oil.

The form of packaging was as follows: RO-85 model can with 65.15 +-0.2 mm in diameter and 34.30+-0.1 mm in height.

To evaluate the effect of sterilization on the bioactive component, in the samples of canned tuna, we compared the initial amount of CLA in the oil with the amount present in the covering oil and the piece of tuna after the sterilization process.

Thus, in this report, we define the means of coverage as the oil remaining in the can and not absorbed by the tuna once processing has taken place.

The influence of this process of sterilization and preservation of the new product on the stability of the bioactive isomers of CLA in tuna was determined so as to ensure the presence of the bioactive components in the final product, for which the necessary analytical methodology has been perfected as detailed below.

Characterization of fatty acids by gas chromatography in the coverage medium and tuna after the process of sterilization:

To evaluate the effect of the sterilization process on the functional component in samples of canned tuna, we analyzed the amount present in the covering oil and the tuna after the sterilization process.

The fatty acids were analyzed by gas chromatography.The methyl esters of fatty acids (FAMEs) were prepared according to the methodology of Zabala et al., (2006).The FAMEs were analyzed in a gas chromatograph (Agilent Technologies) with a flame ionization detector (FID). The separation of FAMEs was carried out with an HP-Innowax capillary column (30m x 0.32 mm id x 0.25 [micro]m, Agilent Technologies, Inc. (California, USA.)).The column was maintained at 150 [deg.] C for 1 minute after injection, then the temperature increased to 200 [deg.] C at a rate of 15 [deg.] C / min, then to 250[deg.] at 2 [deg.] C / min, and finally kept at this temperature for 10 min. Helium was used as a gas carrier at a constant flow of 1 ml / min. The injection volume was 1 [micro]l. The bioactive CLA isomers were identified by comparison of retention times obtained by commercial standards, administered by Larodan Fine Chemicals (Malmo, Sweden).

The results show that prior to the sterilization process, 67% of the bioactive isomers of CLA were in the covering oil, while the rest (33% of the bioactive isomers of CLA) were absorbed by the tuna.

After the sterilization process there was a slightly different distribution: 75% of the bio-active isomers of CLA were found in the covering oil, while the remainder, 25% remained in the tuna.

Thus, due to the sterilization process, there is an increase in the content ofbioactive isomers of CLA in the covering oil compared with the content found in the tuna.

Several studies were conducted to evaluate the stability of the active isomers of CLA included in the product by determining the content of each of the bioactive isomers of CLA analyzed by GS-FID chromatography. The trials were replicated which allowed confirmation of the results. As reflected in Figures 2 and 3, the results confirm that the content of the active isomers of CLA per can does not decrease during the storage period.

Consequently, one can consider that the bioactive CLA isomers remain stable and do not undergo oxidative degradation in the food matrix to which they have been incorporated, even after the sterilization process and storage.

## Claims

1. Composition based on conjugated linoleic acid for canned products which includes tuna, characterized as including between 7.5 and 15% of the bioactive isomers of CLA at 50:50 in olive oil.

2. Composition according to claim 1, where the olive oil has a composition of 97% refined olive oil and 3% extra virgin olive oil

3. Composition according to claim 1, where the tuna is Thunnus obesus.

4. Composition according to any previous claims for use as reducing body fat and as a lipid metabolism activator.

## Patentansprüche

1. Zusammensetzung auf der Basis von konjugierter Linolsäure für Konserven, die Thunfisch enthält, **dadurch gekennzeichnet, dass** sie 7,5 bis 15% der bioaktiven Isomere von CLA zu 50:50 in Olivenöl enthält.

2. Zusammensetzung gemäß Anspruch 1, wo das Olivenöl eine Zusammensetzung aus 97% raffiniertem Olivenöl und 3% nativem Olivenöl extra hat.

3. Zusammensetzung gemäß Anspruch 1, wo es sich bei dem Thunfisch um Thunnus obesus handelt.

4. Zusammensetzung gemäß aller vorstehender Ansprüche für den Einsatz zur Körperfettverringerung und als Fettstoffwechselaktivator.

## Revendications

1. Composition sur la base d'acide linoléique conjugué pour des conserves contenant du thon, **caractérisée par le fait, qu'**elle contient de 7,5 à 15% d'isomères bioactives du CLA à raison de 50:50 dans l'huile d'olive.

2. Composition d'après la révendication 1, dont l'huile d'olive à une composition de 97% d'huile raffinée et 3% d'huile d'olive vierge extra.

3. Composition d'après la révendication 1, dont il s'agit du thon Thunnus obesus.

4. Composition d'après une des révendications précédentes pour son utilisation comme réducteur de la graisse corporelle et activateur du métabolisme lipidique.
